(19) Europäisches Patentamt
European Patent Office
Office européen des brevets

(11) EP 1 050 510 B1

(12) **EUROPÄISCHE PATENTSCHRIFT**

(45) Veröffentlichungstag und Bekanntmachung des Hinweises auf die Patenterteilung:
**12.11.2003 Patentblatt 2003/46**

(51) Int Cl.[7]: **C01B 33/20**, B01J 23/06
// C07C41/08, C07C41/54, C07C43/16, C07C43/303

(21) Anmeldenummer: **00116291.6**

(22) Anmeldetag: **02.06.1998**

# (54) Verfahren zur Herstellung eines Hemimorphit-Katalysators

Process for the preparation of a hemimorphite catalyst

Procédé pour la préparation d'un catalyseur hémimorphite

(84) Benannte Vertragsstaaten:
**CH DE FR GB IT LI**

(30) Priorität: **23.06.1997 DE 19726670**

(43) Veröffentlichungstag der Anmeldung:
**08.11.2000 Patentblatt 2000/45**

(62) Dokumentnummer(n) der früheren Anmeldung(en) nach Art. 76 EPÜ:
**98110001.9 / 0 887 330**

(73) Patentinhaber: **BASF AKTIENGESELLSCHAFT**
**67056 Ludwigshafen (DE)**

(72) Erfinder:
- **Teles, Joaquim Henrique, Dr.**
  **67166 Otterstadt (DE)**
- **Rieber, Norbert, Dr.**
  **68259 Mannheim (DE)**
- **Breuer, Klaus, Dr.**
  **67122 Altripp (DE)**
- **Demuth, Dirk, Dr.**
  **68161 Mannheim (DE)**
- **Hibst. Hartmut, Prof. Dr.**
  **69198 Schriesheim (DE)**
- **Hagemeyer, Alfred, Dr.**
  **48431 Rheine (DE)**

(74) Vertreter: **Isenbruck, Günter, Dr. et al**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
**EP-A- 0 165 647** **US-A- 2 521 113**

- **CHEMICAL ABSTRACTS, vol. 119, no. 22, 29. November 1993 (1993-11-29) Columbus, Ohio, US; abstract no. 231586n, H. NAGATA: "Analytical study of the formation of hemimorphite. I. Analysis of the crystallization process by the co-precipitation method" Seite 380; Spalte 2; XP002080417 & ZAIRYO TO KANKYO, Bd. 42, Nr. 4, 1993, Seiten 225-233,**
- **CHEMICAL ABSTRACTS, vol. 72, no. 6, 9. Februar 1970 (1970-02-09) Columbus, Ohio, US; abstract no. 27871h, A. G. MERKULOV: "Low-temperature synthesis of zinc silicates" Seite 519; Spalte 1; XP002080675 & IZV. SIB. OTD. AKAD. NAUK SSSR, SER. KHIM. NAUK, Nr. 4, 1969, Seiten 70-74,**
- **CHEMICAL ABSTRACTS, vol. 69, no. 26, 23. Dezember 1968 (1968-12-23) Columbus, Ohio, US; abstract no. 108210g, L. S. PAKHOIMOVA: "Cadmium silicate and zinc silicate catalysts" Seite 10156; Spalte 1; XP002080416 & SU 220 957 A**
- **CHEMICAL ABSTRACTS, vol. 102, no. 24, 17. Juni 1985 (1985-06-17) Columbus, Ohio, US; abstract no. 206083n, "Zinc silicate" Seite 128; Spalte 1; XP002080676 & JP 60 011219 A (MITSUBISHI ELECTRIC) 21. Januar 1985 (1985-01-21)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 050 510 B1

Printed by Jouve, 75001 PARIS (FR)

## Beschreibung

Die Erfindung betrifft ein Verfahren zur Herstellung eines Katalysators mit der Struktur von Hemimorphit.

Die Addition von Hydroxylgruppen enthaltenden Verbindungen an Alkine bzw. Allene wird fast ausnahmsweise mit homogen gelösten Katalysatoren durchgeführt, z.B. mit Säuren, Basen und Übergangsmetallkomplexen (vgl. Houben-Weyl, Methoden der organischen Chemie, Bd 6/3, S. 233, S. 90, Bd. 5/2a, S. 738, Bd. 6/1d, S. 136 und Bd. 7/a, S. 816).

Die Säurekatalyse ist meistens auf die Addition an aktivierte, elektronenreiche Alkine (wie Acetylenether, R-C≡C-OR', Acetylenthioether, R-C≡C-SR' und Acetylenamine, R-C≡C-NR'$_2$) beschränkt.

Basenkatalysiert (in Gegenwart von KOH oder Alkoholat) können Alkohole in der Flüssigphase auch an nicht aktivierte Alkine addiert werden. Dies ist die gebräuchlichste Methode; allerdings bedarf die Reaktion hoher Temperaturen und Drücke und die Raumzeitausbeute ist relativ gering. Typischerweise benötigt man für die basenkatalysierte Vinylierung eines Alkoholes zwischen 6 und 10 h Verweilzeit bei ca. 160°C und 18 bis 20 bar Druck.

Die Addition kann auch durch Übergangsmetallkomplexe in der Flüssigphase katalysiert werden. Für die Addition von Alkoholen eignen sich insbesondere Quecksilber(II)- oder Gold(I)-Salze, während für die Addition von Carbonsäuren und Phenole Zink- und Cadmiumsalze bevorzugt werden.

Die Addition von Carbonsäuren (insbesondere Essigsäure und Propionsäure) an Acetylen kann auch in der Gasphase in Gegenwart entsprechender Zinkcarboxylate (auch basischer Zinkcarboxylate gemäß CH 239 752) auf oberflächenreichen Trägern als Katalysatoren durchgeführt werden.

In der US 2,521,113 wird ein Verfahren zur Herstellung von Carbonsäurevinylestern beschrieben, wobei eine gasförmige Mischung einer Carbonsäure und Acetylen bei erhöhter Temperatur über einen Katalysator geführt werden, der im wesentlichen aus einem Zink- oder Cadmiumsilikat oder -polysilikat besteht und das Acetylen im Überschuß eingesetzt wird. In einer beispielhaften Ausführungsform wird ein Zinksilikat verwendet, das ein molares Verhältnis $SiO_2$/ZnO von 0,9 aufweist.

In der EP 0 165 647 wird ein Verfahren zur Herstellung eines synthetischen kristallinen Zinksilikats beschrieben, wobei ein mit Säure behandeltes Produkt eines Tonminerals und ein Zinkoxid oder -hydroxid unter hydrothermalen Bedingungen miteinander umgesetzt werden.

H. Nagata (Chemical Abstracts, Vol. 119, No. 22, Abstract No. 231586n) beschreibt die Herstellung von Hemimorphit, wobei kolloidales Zn ($OH_2$) mit einer Lösung von $Na_2$ $SiO_3$ x 9 $H_2O$ kopräzipitiert und der Niederschlag bei 85°Celsius für 1 bis 120 Tage gealtert wird.

JP 60-11219 (Chemical Abstracts, Vol. 102, No. 24, 1985, Abstract No. 206083n) beschreibt die Herstellung von Zinksilikaten die 57,9% Zn und 42,1% Si enthalten. Um eine Kopräzipitation von Zn $(OH)_2$ zu vermeiden, wird $NH_3$ oder ein organisches Amin einer Lösung von Zn $(NO_3)_2$ x 6 $H_2O$ und $Na_2$ $SiO_3$ x 9 $H_2O$ beigegeben.

Merkulov et. al. (Chemical Abstracts, Vol. 72, No. 61970, Abstract No. 27875 1h) beschreibt die Herstellung kristalliner Zinksilikate bei Temperaturen von 90 - 100°C. Bei einem pH von 10 - 12 wir Hemimorphit gebildet, bei einem pH von 6,5 - 8,5 Willemit und Zinksilit bei einem pH von 5 - 6.

Schließlich ist auch bereits die Addition von Methanol an Propin bzw. Propadien in der Gasphase in Gegenwart von Zinkoxid auf Aktivkohle oder Silikagel in DD 265 289 bzw. von Zinknitrat auf Aktivkohle oder Silikagel in DD 267 629 beschrieben.

Alle diese Verfahren des Standes der Technik haben Nachteile. Sie haben entweder nur ein beschränktes Anwendungsgebiet oder benötigen, wie die basenkatalysierte Addition, hohe Drücke und Temperaturen, was zu Sicherheitsproblemen führen kann, oder sie haben nur eine geringe Raumzeitausbeute. Homogen gelöste Übergangsmetallkatalysatoren sind oft nach einer geringen Anzahl von Zyklen desaktiviert und sind außerdem schwierig zurückzuführen. Heterogene Katalysatoren für die Addition an Alkine bzw. Allene sind bisher nur selten beschrieben worden. Zink- bzw. Cadmiumcarboxylate auf Aktivkohle katalysieren lediglich die Addition von Carbonsäuren (z.B. Essigsäure oder Propionsäure) an Acetylen. Der o.g. Katalysator auf der Basis von Zinkoxid auf Aktivkohle oder Silicagel (DD 265 289) ist zwar in der Lage die Addition von Alkoholen (Methanol oder Ethanol) an Propin bzw. Propadien mit guter Selektivität (90 bis 96%) zu katalysieren, die katalytische Aktivität ist aber relativ gering und die benötigten Reaktionstemperaturen und Kontaktzeiten sind hoch. Zinknitrat auf Aktivkohle oder Silicagel ist bei gleicher Temperatur ungefähr eine Größenordnung weniger aktiv als das Zinkoxid und die Selektivität ist viel geringer (max. 70 %).

Die Herstellung von Hemimorphit ist aus der Literatur bekannt. Sie kann unter Normalbedingungen oder hydrothermalen Bedingungen erfolgen.

Hemimorphit-Katalysator

a) Herstellung unter Normalbedingungen

A.G. Merkulov u. B. S. Khristoforov, (Tr. Soveshch. Eksp. Tekh. Mineral. Petrogr., 8th (1971), Meeting Date 1968, 322-8; Editor(s): V. V. Lapin; Publisher: "Nauka", Moscow, USSR) beschreiben die Herstellung verschiedener Zn-Silikate durch eine Reaktion von verschiedenen Zinksalzen (Carbonat, Sulfat, Chlorid, Acetat, Oxid) mit Na-Silikat und Natronlauge in wäßriger Lösung bei Temperaturen von 90 - 100°C und bei Normaldruck. Dabei bilden sich in Abhängigkeit vom eingestellten pH-Wert unterschiedliche Zinksilikate. So entsteht reiner Sauconit mit der Zusammensetzung $Zn_3Si_4O_{10}(OH)_2 \cdot n\ H_2O$ bei einem End-pH-Wert von 5 - 6. Reiner Willemit ($\alpha$-$Zn_2SiO_4$) fällt im pH-Bereich von 6,5 - 8,5 an. Reiner Hemimorphit ($Zn_4Si_2O_7(OH)_2 \cdot H_2O$) kristallisiert dagegen nur im schwach alkalischen Medium bei pH-Werten von größer 10 aus.

In einer anderen Arbeit der genannten Autoren (A. G. Merkulov u. B. S. Khristoforov, Izv. Sib. Otd. Akad. Nauk SSSR, Ser. Khim. Nauk (1969), (4), 70 - 4) wird angegeben, daß reiner Hemimorphit bei der Umsetzung von Zinksalzen mit Natriumsilikat und Natronlauge bei 90 - 100°C und Atmosphärendruck in wäßriger Lösung nur in einem pH-Bereich von 10 - 12 gebildet wird.

Ferner konnten T. Baird, A. G. Cairns Smith und D. S. Snell (Reactivity of Solids, Proc. Int. Symp., 8th (1977), Göteborg, Meeting Date 1976, 337 - 42; Editor(s): J. Wood, O. Lindqvist u. C. Helgesson; Publisher: Plenum Press, New York, N. Y.) große Kristalle von Hemimorphit durch Umsetzung von $Zn(OH)_2$ mit Kieselsäure und LiOH in wäßriger Lösung bei einem pH von 10 herstellen.

Schließlich wurde von H. Nagata, M. Matsunage u. K. Hosokawa (Zairyo-to-Kankyo (1993) 42, 225 - 233) Hemimorphit hergestellt, indem wäßrige Zinksulfat-Lösung mit Natronlauge und wäßriger Natriumsilikat-Lösung bei einem pH von 13 umgesetzt wurde, der erhaltene Niederschlag abgetrennt, ausgewaschen und bei 85°C mindestens 24 Stunden lang gealtert wurde.

b) Hydrothermale Herstellung

Gemäß EP 165 647 kann Hemimorphit aus einem säurebehandelten Tonmineral und Zinkoxid bzw. Zinkhydroxid unter hydrothermalen Bedingungen (170°C, 5h) hergestellt werden. Die Säurevorbehandlung des Tones ist jedoch sehr aufwendig und deshalb ist dieses Verfahren nachteilig.

Gemäß D. M. Roy u. F. A. Mumpton (Econ. Geol. (1956) 51, 432 - 443) kann Hemimorphit ferner durch hydrothermale Umsetzung von Mischungen aus ZnO und $SiO_2$ bei 175 - 200°C gewonnen werden (Zusammensetzung : 3 ZnO + 2 $SiO_2$). Das erhaltene Produkt enthält überwiegend Hemimorphit, ist aber durch Sauconit ($Zn_3Si_4O_{10}(OH)_2 \cdot 4H_2O$) verunreinigt.

Schließlich beschreiben P. Taylor u. D. G. Owen, (Polyhedron (1984) 3(2) 151-155) die hydrothermale Synthese von Hemimorphit durch Umsetzung von ZnO mit $SiO_2$ in wäßriger Lösung bei 150°C. Zur Herstellung von Produkten mit einem hohen Hemimorphit-Anteil waren jedoch lange Reaktionszeiten von mindestens 4 Tagen erforderlich.

Obgleich Hemimorphit-Produkte, die nach den oben beschriebenen bekannten Methoden erhalten wurden, sich als Katalysator für die Addition von Hydroxylgruppen enthaltenden Verbindungen an Alkine oder Alkene sehr gut eignen, ergab sich, daß es wünschenswert war, deren Eigenschaften noch zu verbessern und eine Herstellungsmethode vorzuschlagen, mit deren Hilfe sich Katalysatoren mit reproduzierbar gutem Eigenschaftsprofil herstellen lassen.

Es bestand daher die Aufgabe, ein Verfahren zur Herstellung von Hemimorphit vorzuschlagen, das einfach und rasch durchzuführen ist, und die Herstellung von Katalysatoren mit reproduzierbaren Eigenschaften erlaubt.

Diese Aufgabe wurde erfindungsgemäß gelöst mit einem Verfahren zur Herstellung eines Zinksilikats mit Hemimorphit-Struktur, das dadurch gekennzeichnet ist, daß man eine Alkalisilikat-Suspension mit einer wässrigen Lösung eines Zinksalzes bei Normaldruck und bei Temperaturen von 50°C bis zum Siedepunkt der entstehenden Suspension oder unter hydrothermalen Bedingungen bei Temperaturen vom Siedepunkt der Suspension bis zu 250°C umsetzt, wobei der pH-Wert der wäßrigen Suspension während und nach der Umsetzung im Bereich von 4 bis 9,5 gehalten und ein Atomverhältnis Zn/Si von 1,6 bis 2,5 eingehalten wird.

Demgemäß wurde eine neue Herstellungsmethode sowohl unter Normaldruck als auch unter hydrothemalen Bedingungen gefunden, bei der man ein Alkali- oder Erdalkalisilikat, vorzugsweise Natriumsilikat mit einem Zinksalz, insbesondere Zinknitrat, und einer Base wie Alkali- oder Erdalkalihydroxid, insbesondere Natriumhydroxid in wäßriger Lösung bei pH-Werten von 4 bis 9,5 vorzugsweise 5,5 bis 8 und insbesondere in einem pH-Bereich um den Neutralpunkt, z.B. bei pH 6 bis 7,5, im Falle von Normaldruck bei Temperaturen von 50 bis 100°C, insbesondere 70 bis 100°C und im Falle von hydrothermalen Bedingungen bei Temperaturen von 100 bis 250°C, bevorzugt im Bereich von 100 bis 200°C umgesetzt.

Nach der neuen Herstellungsmethode kann reiner Hemimorphit mit einem Zn/Si-Verhältnis von 2 synthetisiert werden. Es sind aber auch Hemimorphit-Präparate mit bis zu 25 % Unter- oder Überschuß an Zink, entsprechend einem Atomverhältnis Zn:Si von 1,6 bis 2,5 zugänglich. Als Katalysatoren werden Hemimorphite bevorzugt, die 0 bis 20 % Zinküberschuß enthalten. Besonders bevorzugt sind Hemimorphite, die 0 bis 10 % Zinküberschuß enthalten.

Die Hemimorphit-Produkte fallen bei der Synthese als weißer kristalliner Niederschlag in Form einer wäßrigen

Suspension an und müssen durch geeignete Maßnahmen, z.B. Filtration oder Zentrifugieren, von der wäßrigen Lösung getrennt werden. Im Falle der Filtration wird der erhaltene Filterkuchen daraufhin ausgewaschen, um es von löslichen Salzen zu befreien, und anschließend getrocknet. Die Trocknung kann bei Temperaturen bis 600°C erfolgen, wobei der bevorzugte Temperaturbereich 90 bis 450°C umfasst. Thermogravimetrische Untersuchungen haben gezeigt, daß der auskristallisierte Hemimorphit der Zusammensetzung $Zn_4Si_2O_7(OH)_2 \cdot H_2O$ im Temperaturbereich von etwa 100 bis 200°C unter Beibehaltung der Hemimorphit-Struktur zunehmende Anteile seines Kristallwassers verliert, wobei Hemimorphit-Präparate der Zusammensetzung $Zn_4Si_2O_7(OH)_2 \cdot xH_2O$ mit x kleiner als 1 resultieren, wobei x mit steigender Temperatur abnimmt. Trocknet man in einem höheren Temperaturbereich von etwa 200 bis 600°C, so werden zusätzlich, ebenfalls unter Beibehaltung der Hemimorphit-Struktur, die im Hemimorphit enthaltenen $OH^-$-Ionen in $O^{2-}$-Ionen und abgespaltenes $H_2O$ umgewandelt ($2\,OH^- \rightarrow H_2O + O^{2-}$), wobei Hemimorphit-Präparate der Zusammensetzung $Zn_4Si_2O_7(OH)_{2-2y}O_y$ mit y=0 bis 1 resultieren, wobei y mit steigender Temperatur zunimmt.

Die nach der Trocknung bei Temperaturen bis 600°C, bevorzugt bei 90 bis 450°C erhaltenen Hemimorphit-Präparate der Zusammensetzung $Zn_4Si_2O_7(OH)_{2-2y}O_y \cdot xH_2O$, wobei x und y Werte von 0 bis 1 umfasst, werden anschließend üblicherweise mit den gebräuchlichen Formgebungsverfahren, z B. Tablettieren, Verstrangen oder als Schalenkatalysatoren auf Steatitkugeln zu katalytischen Formkörpern verarbeitet. Einzelheiten werden in den Beispielen beschrieben.

Die erfindungsgemäßen Zinksilikate können mit bis zu 80, vorzugsweise 50 und insbesondere bis zu 20 Molprozent noch mit weiteren Metallen dotiert sein ausgewählt aus der Gruppe (A) bestehend aus Beryllium, Magnesium, Calzium, Strontium, Barium, Mangan, Eisen, Kobalt, Nickel, Kupfer, Cadmium und Quecksilber und der Gruppe (B), bestehend aus Titan, Zirkonium, Hafnium, Germanium, Zinn und Blei, wobei die Elemente der Gruppe (A) teilweise Zink und die Elemente der Gruppe (B) teilweise Silizium in der Formel VI ersetzen.

Als Katalysatoren mit der Struktur des Hemimorphits können auch solche verwendet werden, bei denen das Verhältnis Zink zu Silizium nicht stöchiometrisch ist, vielmehr einen bis zu 25%igen Unter- oder Überschuß an Zink aufweisen, entsprechend einem Atomverhältnis Zn:Si von 1,6 bis 2,5. Bevorzugt sind Hemimorphit-Katalysatoren, die 0 bis 20 %, insbesondere 0 bis 10 % Zinküberschuß aufweisen.

Wesentlich für die erfindungsgemäße Reaktion ist die Verwendung eines heterogenen Katalysators, der zumindest überwiegend als Aktivkomponente Zinksilikat mit der Struktur des Hemimorphits der Formel $Zn_4Si_2O_7(OH)_{2-y}O_y \cdot xH_2O$ enthält, wobei x und y für die Werte 0 bis 1 stehen.

Der Hemimorphit-Katalysator eignet sich für eine Verwendung in einem Verfahren zur Herstellung von Verbindungen der Formeln I bzw. II

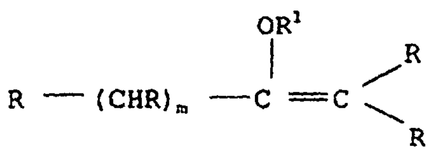

I

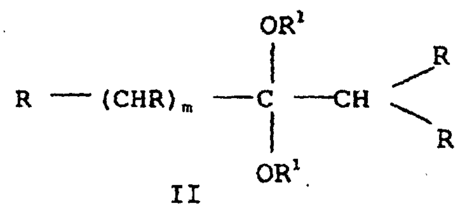

II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können, die Reste R unabhängig voneinander für Wasserstoff, oder aliphatische, cycloaliphatische, araliphatische, aromatische oder heterocyclische Reste stehen, die unter Bildung eines Ringes miteinander verbunden sein können, und m für 0 oder 1 steht, durch Addition von Verbindungen der Formel III

$$R^1OH \qquad \text{III}$$

an Acetylene oder Allene der Formeln IV bzw. V

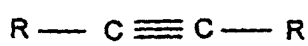

IV

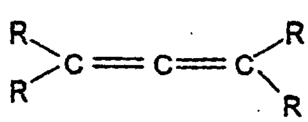

V

wobei $R^1$ und R die oben angegebene Bedeutung haben, in der Gasphase bei erhöhter Temperatur.

Als Edukte für die Umsetzung kommen beliebige Alkine oder Allene oder Gemische davon in Betracht. In der Regel wird man jedoch technisch leicht zugängliche Acetylene und Allene mit 2 bis 8 C-Atomen, bzw. 3 bis 8 C-Atomen verwenden. Besonders bevorzugt sind Propin und Allen und insbesondere Kohlenwasserstoffströme, die diese enthalten.

Die Hydroxylgruppen enthaltende Verbindung $R^1OH$ kann Wasser, ein beliebiger Alkohol, ein Phenol oder eine Carbonsäure sein. Im allgemeinen kommen vor allem Alkohole, besonders Alkanole mit 1 bis 16 C-Atomen, einkernige Phenole und niedermolekulare Carbonsäuren, z.B. mit 1 bis 16 C-Atomen, in Betracht. Besonders bevorzugt werden niedere Alkohole und insbesondere Methanol verwendet.

Die Addition der Hydroxylgruppen enthaltenden Verbindungen erfolgt in Gegenwart des heterogen vorliegenden Katalysators in der Gasphase entweder über einem Festbett oder in einem Wirbelbett bei Temperaturen von 50 bis 400°C, vorzugsweise 100 bis 250°C und besonders bevorzugt 120 bis 200°C und Drücken von 0,1 bis 100 bar, insbesondere 0,8 bis 20 bar (alle Drücke bezogen auf Summe der Partialdrücke der Edukte).

Gegebenenfalls kann das Reaktionsgemisch aus Gründen der Betriebssicherheit und der besseren Wärmeabfuhr mit Inertgasen wie Stickstoff, Argon, niedermolekularen Alkanen oder Olefinen verdünnt werden.

Das molare Verhältnis zwischen der Hydroxylgruppen enthaltenden Komponente und Alkin bzw. Allen kann zwischen 0,01 und 100 liegen. Bevorzugt wird der Bereich zwischen 0,1 und 5, und besonders bevorzugt wird der Bereich zwischen 0,7 und 1,3 gewählt.

Durch die Reaktionsbedingungen kann die Selektivität der Reaktion bezüglich der Mono- bzw. Diadditionsprodukte gesteuert werden. Niedrige Verhältnisse zwischen der Hydroxylgruppen enthaltenden Komponente und Alkin bzw. Allen sowie hohe Temperaturen und niedrige Partialdrücke der Reaktanden führen zur bevorzugten Bildung der Monoadditionsprodukte während hohe Verhältnisse zwischen der Hydroxylgruppen enthaltenden Komponente und Alkin bzw. Allen sowie niedrige Temperaturen und hohe Partialdrücke der Reaktanden die Bildung der Bisadditionsprodukte begünstigen. Beispielsweise kann aus Propin oder Allen mit Methanol je nach Reaktionsbedingungen selektiv 2-Methoxypropen oder 2,2-Dimethoxypropan gebildet werden.

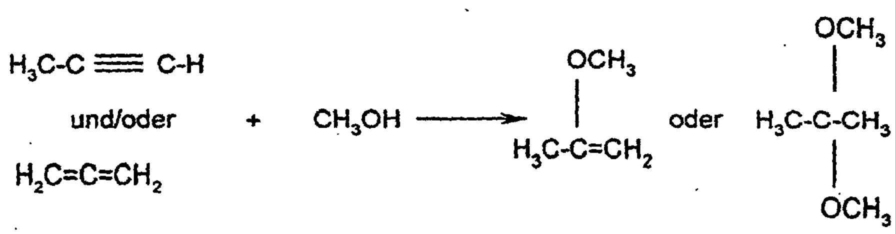

Für die Charakterisierung der Katalysatorproben (frische als auch Ausbauproben) wurden Standardmethoden verwendet. Die gemessene BET-Oberfläche liegt in der Regel zwischen 50 und 500 $m^2/g$. Bevorzugt werden Katalysatoren mit BET-Oberflächen zwischen 80 und 400 $m^2/g$ verwendet. Weiterhin wurden die mit dem neuen Herstellungsverfahren erhaltenen Proben mittels Pulverröntgendiffraktometrie (XRD) untersucht

Allgemeine Umsetzungsbedingungen

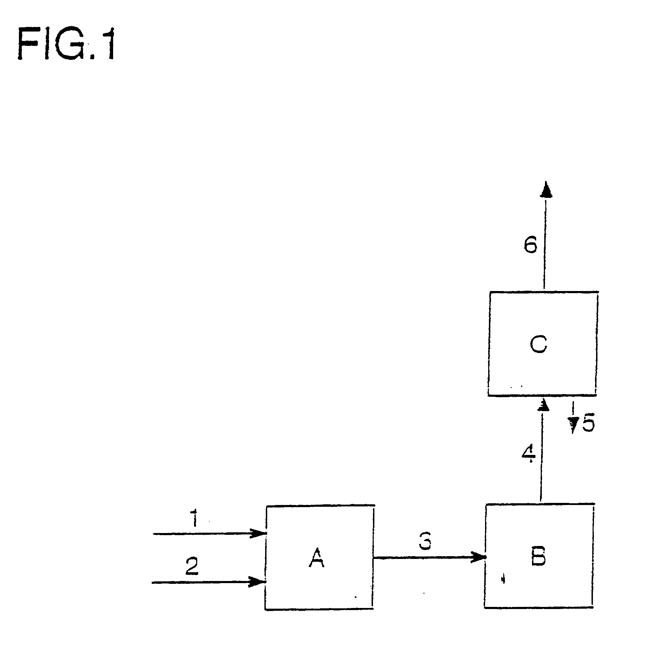

Die katalytischen Umsetzungen gemäß Fig 1. wurden in einem gradientenfreien CSTR (Continuously Stirred Tank Reactor) (A) mit einem Volumen von 1740 ml und einem Katalysatorvolumen von ca. 90 ml, modifiziert für heterogene Gasphasenreaktionen durchgeführt. Der Reaktor hatte einen Innendurchmesser von ca. 108 mm und eine Höhe von ca. 200 mm und wurde mittels einer an der Innenwand angebrachten elektrischen Heizspirale beheizt. In der Mitte des Reaktors war ein kleiner Zylinder aus Metall angebracht (∅ ca. 64 mm, Höhe ca 150 mm), der auf halbe Höhe (ca. 85 mm unterhalb der Oberkante) mit einem Drahtgitter versehen war. Auf dieses Drahtgitter wurde der Katalysator locker aufgeschüttet. Auf dem Deckel des Reaktors war eine flache (Ø ca. 100 mm, Höhe ca. 20mm) Turbine angebracht, die mit 1500-2000 Upm angetrieben wurde. An der Reaktorachse waren auf verschiedenen Höhen insgesamt 6 Thermoelemente zur Temperaturkontrolle angebracht.

Die Edukte wurden unter Druck mittels HPLC-Pumpen dosiert, kurz vor dem Reaktor gemischt und in den Reaktorraum entspannt. Das Alkin bzw. Allen (1 in Fig 1) wurden entweder in reiner Form zudosiert oder als Gemisch mit anderen inerten Komponenten verdünnt. In dem Fall von Propin und Allen wurde ein Gemisch mit anderen Kohlenwasserstoffen eingesetzt (Zusammensetzung: 30-43 Vol% Propin, 16-20 Vol% Allen, 20-45 Vol% Propen, 5-10 Vol% Isobutan und 2-6 Vol% Propan als Hauptkomponenten; alle anderen Komponenten unter 1 %. Dieses Gemisch wurde durch Destillation aus einem Seitenstrom eines Steamcrackers gewonnen).

Der Alkoholkomponente (2 in Fig 1) wurden ca. 10 Gew.-% Cyclohexan als interner Standard für die GC-Analytik zudosiert.

Die Reaktion wurde isotherm bei Temperaturen von 120 bis 300°C und einer Zulaufrate von 0,5 bis 10 mmol/min Propin und/oder Allen und 0,5 bis 20 mmol/min MeOH durchgeführt. Der Reaktionsdruck betrug 1,1 bis 3,5 bar (absolut).

Die Gesamtgasmenge, bestehend aus Edukten, Inertgas und internem Standard, betrug in der Regel zwischen 4 und 60 N1/h. Die GHSV (gas hourly space velocity), die definiert ist als

$$GHSV = Gasvolumen\ [N1/h]/Katalysatorvolumen\ [l]$$

betrug zwischen 80 und 1200 $h^{-1}$. Die LHSV (liquid hourly space velocity), die definiert ist als

LHSV = Flüssigkeitsvolumen [N1/h]/Katalysatorvolumen [l] (hier gefördertes Volumen an

Propin und MeOH Volumen)

betrug zwischen 0,2 und 3 $h^{-1}$. Die Verweilzeit, definiert als Quotient aus dem Katalysatorvolumen [l] und der Gasmenge [Nl/s] betrug zwischen 3 und 40 s.

Die Reaktionsgase wurden nach Verlassen des Reaktors über eine beheizte Transferleitung (3) zu einem On-line Gaschromatographen (B) geführt und dort alle 2 h analysiert. Danach wurde der Gasstrom einer partiellen Kondensation (C) unterworfen und der bei Raumtemperatur nicht kondensierbare Anteil (6) wurde in regelmäßigen Abständen (ca. 12 h) mittels Off-line GC analysiert. Das Kondensat (5) wurde ebenfalls gesammelt und mittels Off-line GC analysiert.

Die erfindungsgemäß erhältlichen Enolether der Formel I und die Dialkoxyverbindungen der Formel II sind wertvolle Zwischenprodukte zur Herstellung von Wirkstoffen und Riechstoffen. Insbesondere die Enolether sind begehrte Ausgangsstoffe z.B. zur Herstellung von $\gamma,\delta$-ungesättigten Ketonen als Vorprodukte für die Herstellung von Isophytol.

Will man vor allem die Enolether gewinnen, kann man in an sich bekannter Weise die Verbindungen der Formel II durch Abspaltung von einem Mol $R^1OH$ in die entsprechenden Enolether der Formel I überführen. Dafür existieren zahlreiche aus DE-A-35 35 128, DE-A- 37 22 891, DE-A-38 04 162, Chemical Abstracts, Vol. 94 (19); 156 241 fund DE-A-19544450 bekannte Verfahren.

Beispiel 1

Herstellung von $Zn_4Si_2O_7(OH)_2 \cdot H_2O$ mit Zn/Si =2 (stöchiometrischer Hemimorphit) und katalytische Umsetzung

In einem 8-l-Rührbehälter stellte man aus 4,5 l vollentsalztem Wasser und 145,1 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 1,5 Mol $SiO_2$ und 0,89 Mol Na her. Ferner wurden 910,7 g $Zn(NO_3)_2 \cdot 6\ H_2O$ (98 %ig) in 2,25 l vollentsalztem Wasser bei Raumtemperatur gelöst, wobei eine Lösung B mit 3 Mol Zn und 6 Mol $NO_3$ erhalten wurde. Schließlich wurde eine wäßrige Lösung aus 204,4 g NaOH in 225 ml vollentsalztem Wasser hergestellt, wobei eine Lösung C mit einem Na-Gehalt von 5,11 Mol erhalten wurde. Nun wurden die Lösungen B und C bei Raumtemperatur in die Suspension A gegeben, wobei sich eine milchige Suspension D mit folgenden Elementanteilen ergab: Zn-Gehalt = 3 Mol, Si-Gehalt = 1,5 Mol, Na-Gehalt = 6 Mol, $NO_3$-Gehalt = 6 Mol. Der pH-Wert der erhaltenen Suspension D betrug 7,1. Die Suspension D wurde auf 90°C aufgeheizt und bei dieser Temperatur 24 Stunden lang mit einer Umdrehungszahl von 200 Upm gerührt. Die Suspension wurde anschließend auf Raumtemperatur abgekühlt und ein End-pH-Wert von 7,0 gemessen. Der auskristallisierte weiße Niederschlag wurde abfiltriert und mit vollentsalztem Wasser Na-frei gewaschen und der anfallende Filterkuchen bei 90°C in einem Trockenschrank getrocknet. Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit die Herstellung von $Zn_4Si_2O_7(OH)_2 \cdot H_2O$ anzeigte. Die nach BET bestimmte spezifische Oberfläche des erhaltenen Pulvers betrug 30 $m^2/g$.

Zur Herstellung eines Katalysators wurde das noch feuchte Pulver direkt zu Strängen verpresst (∅ = 3 mm, Preßdruck = 50 bar), die anschließend 16 h bei 120°C getrocknet wurden. Der fertigen Katalysator hatte eine BET-Oberfläche von 26 $m^2/g$, eine Härte von 6 N/Formkörper.

In die unter (a) beschriebene Apparatur wurden ca. 90 ml des Katalysators eingefüllt und das Propin/Allen-Gemisch (ca. 63 Vol%-ig, 2,8 mmol/min) und Methanol (4,7 mmol/min; Gesamtzulauf mit Inerten: 9,4 mmol/min; Verhältnis MeOH/(Propin+Allen)=1,68) wurden dann mittels HPLC-Pumpen zudosiert. Die Reaktionstemperatur betrug 200°C und der Druck 1,25 bar (Partialdruck der Edukte: 1,0 bar). Es wurden von Anfang an (d.h. der Katalysator hat keine Formierungszeit) folgende Selektivitäten beobachtet: 2-Methoxypropen = 91 %; 2,2-Dimethoxypropan = 5 %; Aceton = 1 %; cis und trans 1-Methoxypropen = 3 %. Der Propin/Allen-Umsatz betrug 48 %. Für den Katalysator wurden nach dem Ausbau die folgenden Werte ermittelt: BET = 25 $m^2/g$, Härte = 6 N/Formkörper.

Beispiel 2

Katalysatorherstellung von Hemimorphit mit Zn/Si = 2,2 und katalytische Umsetzung

In einem 6-l-Rührbehälter wurde aus 3,0 l vollentsalztem Wasser und 96,8 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 1,0 Mol $SiO_2$ und 0,59 Mol Na und aus 667,8 g $Zn(NO_3)_2 \cdot 6\ H_2O$ (98 %-ig) in 1,5 l vollentsalztem Wasser bei Raumtemperatur eine Lösung B mit 2,2 Mol Zn und 4,4 Mol $NO_3$ sowie eine wäßrige Lösung C mit einem Na-Gehalt von 3,81 Mol aus 152,3 g NaOH in 400 ml vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 2,2 Mol, Si-Gehalt = 1 Mol, Na-Gehalt = 4,4 Mol, $NO_3$-Gehalt = 4,4 Mol. Der pH-Wert der erhaltenen Suspension D betrug 7,2. Die Suspension D wurde auf 90°C erhitzt und bei dieser Temperatur 24 Stunden lang mit einer Umdrehungszahl von 200 Upm gerührt. Nach Abkühlen der Suspension auf Raumtemperatur wurde ein End-pH-Wert von 7,0 gemessen. Der angefallene weiße Niederschlag wurde abfiltriert, mit vollentsalztem Wasser Na-frei gewaschen und der erhaltene Filterkuchen bei 90°C in einem Trockenschrank getrocknet.

Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit die Herstellung von $Zn_4Si_2O_7(OH)_2 \cdot H_2O$ anzeigte. Die nach BET bestimmte spezifische Oberfläche des erhaltenen Pulvers betrug 60 $m^2/g$.

650 g des Pulvers wurden mit 20,2 g Magnesiumstearat (Merck) vermischt und zu 20 mm Tabletten gepreßt. Diese Tabletten wurden zu Splitt (<0,5 mm) zerdrückt und der Split zu Ringlochtabletten (5 x 5 x 2 mm) verarbeitet. Die Tabletten wurden dann 10 Stunden bei 350°C kalziniert. Der fertige Katalysator hatte eine BET-Oberfläche von 44 $m^2/g$, und eine Härte von 44 N/Formkörper.

In die unter (a) beschriebene Apparatur wurden ca. 90 ml des Katalysators eingefüllt und das Propin/Allen-Gemisch (ca. 60 Vol%-ig, 2,8 mmol/min) und Methanol (2,0 mmol/min; Gesamtzulauf mit Inerten: 6,8 mmol/min; Verhältnis MeOH/(Propin+Allen)= 0,72) mittels HPLC-Pumpen zudosiert. Die Reaktionstemperatur betrug 170°C und der Druck 1,1 bar (abs) (Partialdruck der Edukte: 0,8 bar). Es wurden von Anfang an (d.h. der Katalysator hatte keine Formierungszeit) folgende Selektivitäten beobachtet: 2-Methoxypropen = 90 %; 2,2-Dimethoxypropan = 3 %; Aceton = 2 %; cis und trans 1-Methoxypropen = 2 %. Der Propin/Allen-Umsatz betrug 71 % und der Methanol-Umsatz 98 %.

Für den Katalysator wurden nach dem Ausbau die folgenden physikalischen Werte ermittelt: BET 44 = $m^2/g$; Härte = 12 N/Formkörper.

Beispiel 3

Hydrothermale Herstellung von $Zn_4Si_2O_7(OH)_2 \cdot H_2O$ mit Zn/Si = 2 und katalytische Umsetzung

In einem 2-l-Rührbehälter wurde aus 200 ml vollentsalztem Wasser und 19,35 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 0,2 Mol $SiO_2$ und 0,12 Mol Na und aus 121,4 g $Zn(NO_3)_2 \cdot 6\ H_2O$ (98 %-ig) in 200 ml vollentsalztem Wasser bei Raumtemperatur eine Lösung B mit 0,4 Mol Zn und 0,8 Mol $NO_3$ sowie eine wäßrige Lösung C mit einem Na-Gehalt von 0,68 Mol aus 27,2 g NaOH in 100 ml vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 0,4 Mol, Si-Gehalt = 0,2 Mol, Na-Gehalt = 0,8 Mol, $NO_3$-Gehalt = 0,8 Mol. Der pH-Wert der erhaltenen Suspension D betrug 7,0. Die Suspension D wurde in einem Autoklaven aus einer Hastelloy-Legierung mit einem Innenvolumen von 1,2l auf 150°C erhitzt und 6 Stunden lang bei dieser Temperatur mit einer Umdrehungszahl von 200 Upm gerührt, wobei ein Überdruck von 7 bar gemessen wurde. Nach Abkühlen der erhaltenen Suspension auf Raumtemperatur wurde ein End-pH-Wert von 7,2 gemessen. Der auskristallisierte weiße Niederschlag wurde abfiltriert und mit vollentsalztem Wasser Na-frei gewaschen und der anfallende Filterkuchen bei 90°C in einem Trockenschrank getrocknet.

Das Röntgenpulverdiagramm des getrockneten weißen Pulvers entsprach dem der Karteikarte 5-0555 der JCPDS-ICDD-Kartei (1995) und beweist die Herstellung von $Zn_4Si_2O_7(OH)_2 \cdot H_2O$. Die nach BET bestimmte spezifische

Oberfläche des erhaltenen Pulvers betrug 28,7 $m^2$/g.

150 g Steatitkugeln (Ø = 2 mm) wurden auf einem Drehteller mit 19,2 g einer 1,25 Gew.-%-igen wäßrigen Lösung von Walocel® (Fa. Wolff, Walsrode), 5,60 g des $Zn_4Si_2O_7(OH)_2 \cdot H_2O$-Pulvers und 8,70 g Zink-Pulver beschichtet Nach dem Trocknen (4 h bei 120°C) erhielt man einen Schalenkatalysator.

In die unter (a) beschriebenen Apparatur wurden ca. 90 ml des Katalysators eingefüllt und das Propin/Allen-Gemisch (ca. 61 Vol%-ig, 3,0 mmol/min) und Methanol (4,7 mmol/min; Gesamtzulauf mit Inerten: 9,7 mmol/min; Verhältnis MeOH/(Propin + Allen) = 1,59) mittels HPLC-Pumpen zudosiert. Die Reaktionstemperatur betrug 200°C und der Druck 1,25 bar (abs) (Partialdruck der Edukte: 1,0 bar). Der Propin/Allen-Umsatz war von Anfang an konstant und betrug ca. 17 %. Als Produkte bildeten sich (Selektivitäten in Klammern): 2-Methoxypropen (90 %), 2,2-Dimethoxypropan (3 %) und cis- und trans-1-Methoxypropen (6 %).

Beispiel 4 (Vergleichsbeispiel)

Katalysatorherstellung von b-$Zn_2SiO_4$ (Zn/Si = 2) und katalytische Umsetzung

100 g des gemäß Beispiel 1 hergestellten Hemimorphits wurden in einem Temperofen 1 Stunde lang bei 700°C erhitzt. Das erhaltene Pulver wurde röntgenographisch untersucht, wobei sich ein Röntgenpulverdiagramm ergab, das dem der Karteikarte 14-0653 der JCPDS-ICDD-Kartei (1995) voll entsprach und damit die phasenreine Bildung von b-$Zn_2SiO_4$ anzeigte. Die nach BET bestimmte spezifische Oberfläche des erhaltenen weißen Pulvers betrug 25 $m^2$/g.

150 g Steatitkugeln (Ø 2 mm) wurden auf einem Drehteller mit 17,2 g einer 1,25 Gew.-%-igen wäßrigen Lösung von Walocel® (Fa. Wolff, Walsrode), 5,50 g des $\beta$-$Zn_2SiO_4$-Pulvers und 5,50 g Zink- Pulver beschichtet. Nach dem Trocknen (4 h bei 120°C) erhielt man einen Schalenkatalysator.

In die unter (a) beschriebene Apparatur wurden ca. 90 ml des Katalysators eingefüllt und das Propin/Allen-Gemisch (ca. 63 Vol%-ig, 3,1 mmol/min) und Methanol (3,9 mmol/min; Gesamtzulauf mit Inerten: 9,0 mmol/min; Verhältnis MeOH/(Propin + Allen) = 1,25) mittels HPLC-Pumpen zudosiert. Die Reaktionstemperatur betrug 200°C und der Druck 1,2 bar (abs). Der Propin/Allen-Umsatz war kleiner 1 % und stieg auch nicht mit der Zeit an.

Beispiel 5 (Vergleichsbeispiel)

Katalysatorherstellung von $\alpha$-$Zn_2SiO_4$(Willemit) und katalytische Umsetzung

100 g des gemäß Beispiel 1 hergestellten Hemimorphits wurden in einem Temperofen 1 Stunde lang bei 800°C erhitzt. Das erhaltene Pulver wurde röntgenographisch untersucht, wobei sich ein Röntgenpulverdiagramm ergab, das dem der Karteikarte 37-1485 der JCPDS-ICDD-Kartei (1995) voll entsprach und damit die einphasige Herstellung von $\alpha$-$Zn_2SiO_4$ (Willemit) anzeigte. Die nach BET bestimmte spezifische Oberfläche des erhaltenen weißen Pulvers betrug 18 $m^2$/g.

Das Pulver wurde mit 2 Gew.-% Graphit und 3 Gew.-% Magnesiumstearat gemischt und zu 3 x 5 mm Tabletten gepreßt. Diese Tabletten wiesen eine BET-Oberfläche von 0,6 $m^2$/g auf.

In die unter (a) beschriebene Apparatur wurden ca. 90 ml des Katalysators eingefüllt und das Propin/Allen-Gemisch (ca. 62 Vol%ig, 3,3 mmol/min) und Methanol (4,0 mmol/min; Gesamtzulauf mit Inerten: 9,5 mmol/min; Verhältnis MeOH/(Propin + Allen) = 1,21) mittels HPLC-Pumpen zudosiert. Die Reaktionstemperatur betrug 200°C und der Druck 1,25 bar (abs). Der Propin/Allen-Umsatz war kleiner 1 % und stieg auch nicht mit der Zeit an.

Beispiel 6 (Vergleichsbeispiel)

Katalysatorherstellung von $Zn_3Si_4O_{10}(OH)_2 \cdot 4\ H_2O$ (Sauconit) und katalytische Umsetzung

In einem 6-l-Rührbehälter wurde aus 3l vollentsalztem Wasser und 580,5 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 6 Mol $SiO_2$ und 3,56 Mol Na und aus 607,1 g $Zn(NO_3)_2 \cdot 6\ H_2O$ (98 %-ig) in 1,51 vollentsalztem Wasser bei Raumtemperatur eine Lösung B mit 2 Mol Zn und 4 Mol $NO_3$, sowie eine wäßrige Lösung C mit einem Na-Gehalt von 0,44 Mol aus 17,6 g NaOH in 200 ml vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 2 Mol, Si-Gehalt = 6 Mol, Na-Gehalt = 4,0 Mol, $NO_3$-Gehalt = 4 Mol. Der pH-Wert der erhaltenen Suspension D betrug 6,8. Die Suspension D wurde auf 90°C erhitzt und bei dieser Temperatur 24 Stunden lang mit einer Umdrehungszahl von 200 Upm gerührt. Nach Abkühlen der Suspension auf Raumtemperatur wurde ein End-pH-Wert von 6,9 gemessen. Der angefallene weiße Niederschlag wurde abfiltriert und mit vollentsalztem

Wasser Na-frei gewaschen und der erhaltene Filterkuchen bei 90°C in einem Trockenschrank getrocknet.

Das Röntgenpulverdiagramm des getrockneten Pulvers ergab, das es der Karteikarte 29-1393 der JCPDS-ICDD-Kartei (1995) und damit $Zn_3Si_4O_{10}(OH)_2 \cdot 4\ H_2O$ (Sauconit) entspricht. Die nach BET bestimmte spezifische Oberfläche des erhaltenen weißen Pulvers betrug 216 $m^2$/g.

150 g Steatit-Kugeln (Ø = 2 mm) wurden auf dem Drehteller mit 17,5 g einer 1,25 Gew.-%-igen wäßrigen Lösung von Walocel® (Fa. Wolff, Walsrode) und einer Mischung aus 10,2 g Zink-Pulver und 6,9 g Sauconit beschichtet. Der Katalysator wurde dann 4 h bei 120°C an der Luft getrocknet.

In der oben beschriebenen Apparatur wurden ca. 90 ml des Katalysators eingefüllt. Das Propin/Allen-Gemisch (ca. 63 Vol%-ig, 3,8 mmol/min und Methanol (4.7 mmol/min; Gesamtzulauf mit Inerten: 11,0 mmol/min; Verhältnis MeOH/(Propin + Allen) = 1,25) und Methanol wurden dann mittels HPLC-Pumpen zudosiert. Die Reaktionstemperatur betrug 200°C und der Druck 1,25 bar (abs). Der Propin/Allen-Umsatz war kleiner 1 % und stieg auch nicht mit der Zeit an.

## Patentansprüche

1. Verfahren zur Herstellung eines Zinksilikats mit Hemimorphit-Struktur, in dem man eine Alkalisilikat-Suspension mit einer wässrigen Lösung eines Zinksalzes bei Normaldruck und bei Temperaturen von 50°C bis zum Siedepunkt der entstehenden Suspension oder unter hydrothermalen Bedingungen bei Temperaturen vom Siedepunkt der Suspension bis zu 250°C umsetzt, **dadurch gekennzeichnet, daß** der pH-Wert der wäßrigen Suspension während und nach der Umsetzung im Bereich von 4 bis 9,5 gehalten und ein Atomverhältnis Zn/Si von 1,6 bis 2,5 eingehalten wird.

2. Verfahren zur Herstellung eines für das Verfahren gemäß Anspruch 1 geeigneten Zinksilikats mit Hemimorphit-Struktur und der Zusammensetzung $Zn_4Si_2O_7(OH)_{2-2y}O_y \cdot xH_2O$, wobei x und y für die Werte 0 bis 1 stehen **dadurch gekennzeichnet, daß** man ein gemäß dem Verfahren des Anspruchs 1 erhältliches Zinksilikat mit Hemimorphit-Struktur einer thermischen Nachbehandlung bei Temperaturen von 70 bis 600°C unterwirft.

3. Verfahren gemäß Anspruch 1, **dadurch gekennzeichnet, daß** man für den pH-Wert den Bereich von 5,5 bis 8 einhält.

4. Verfahren gemäß Anspruch 1, **dadurch gekennzeichnet, daß** man für den pH-Wert den Bereich von 6 bis 7,5 einhält.

5. Verfahren gemäß Anspruch 1, **dadurch gekennzeichnet, daß** man bei der Umsetzung weitere Salze von Elementen

   der Gruppe A, bestehend aus Beryllium, Magnesium, Calcium, Strontium, Barium, Mangan, Eisen, Kobalt, Nickel, Kupfer, Cadmium und Quecksilber, und

   der Gruppe B, bestehend aus Titan, Zirkon, Hafnium, Germanium, Zinn und Blei,

   in Mengen von 0 bis 20 Mol-% der Gruppe A, bezogen auf Zink, und 0 bis 20 Mol-% der Gruppe B, bezogen auf Silicium, mitverwendet.

## Claims

1. A process for preparing a zinc silicate having a hemimorphite structure by reacting an alkaline metal silicate suspension with an aqueous solution of a zinc salt at atmospheric pressure and a temperature in the range from 50°C to the boiling point of the suspension formed or under hydrothermal conditions at a temperature from the boiling point of the suspension to 250°C, wherein the pH of the aqueous suspension is kept in the range from 4 to 9.5 during and after the reaction and the Zn/Si atomic ratio is from 1.6 to 2.5.

2. A process for preparing a zinc silicate having a hemimorphite structure and the composition $Zn_4Si_2O_7(OH)_{2-2y}O_yxH_2O$, where x and y are from 0 to 1, which comprises subjecting a zinc silicate having a hemimorphite structure obtainable by the process of claim 1 to a thermal after-treatment at from 70 to 600°C.

3. A process as claimed in claim 1, wherein the pH is kept in the range from 5.5 to 8.

4. A process as claimed in claim 1, wherein the pH is kept in the range from 6 to 7.5.

5. A process as claimed in claim 1, wherein further salts of elements

of group A consisting of beryllium, magnesium, calcium, strontium, barium, manganese, iron, cobalt, nickel, copper, cadmium and mercury, and

of group B consisting of titanium, zirconium, hafnium, germanium, tin and lead

are concomitantly used in the reaction in amounts of from 0 to 20 mol% of group A, based on zinc, and from 0 to 20 mol% of group B, based on silicon.

## Revendications

1. Procédé de préparation d'un silicate de zinc de structure hémimorphique, en faisant réagir une suspension de silicate de métal alcalin avec une solution aqueuse d'un sel de zinc sous la pression normale et à une température supérieure allant de 50°C jusqu'au point d'ébullition de la suspension formée ou dans des conditions hydrothermiques à une température allant du point d'ébullition de la suspension jusqu'à 250°C, **caractérisé en ce que** le pH de la suspension aqueuse est maintenu, pendant et après la réaction, à une valeur allant de 4 à 9,5, et que l'on maintient un rapport atomique Zn/Si allant de 1,6 à 2,5.

2. Procédé de préparation d'un silicate de zinc de structure hémimorphique approprié pour le procédé selon la revendication 1 et de composition
$Zn_4Si_2O_7(OH)_{2-2y}O_y.xH_2O$, dans laquelle x et y valent 0 à 1, caracétrisé en ce que l'on soumet un silicate de zinc de structure hémimorphique, pouvant être obtenu selon le procédé de la revendication 1, à un traitement thermique ultérieur à une température allant de 70°C à 600°C.

3. Procédé selon la revendication 1, **caractérisé en ce que** l'on maintient le pH de 5,5 à 8.

4. Procédé selon la revendication 1, **caractérisé en ce que** l'on maintient le pH de 6 à 7,5.

5. Procédé selon la revendication 1, **caractérisé en ce que** l'on utilise conjointement, lors de la réaction, d'autres sels des éléments

du groupe A, formé par le béryllium, le magnésium, le calcium, le strontium, le baryum, le manganèse, le fer, le cobalt, le nickel, le cuivre, le cadmium et le mercure, et
du groupe B formé par le titane, le zirconium, l'hafnium, le germanium, l'étain et le plomb,
à raison de 0% à 20% en moles du groupe A par rapport au zinc et de 0% à 20% en moles du groupe B par rapport au silicium.

FIG.1